**Europäisches Patentamt**

(19) **European Patent Office**

**Office européen des brevets**

(11) Veröffentlichungsnummer: **0 027 624**
**B1**

(12) # EUROPÄISCHE PATENTSCHRIFT

(45) Veröffentlichungstag der Patentschrift:
**16.03.83**

(21) Anmeldenummer: **80106215.9**

(22) Anmeldetag: **13.10.80**

(51) Int. Cl.³: **A 01 N 37/22,** A 61 K 31/165

(54) **Präparat zur Bekämpfung des Kannibalismus der Schweine.**

(30) Priorität: **22.10.79 DE 2942581**

(43) Veröffentlichungstag der Anmeldung:
**29.04.81 Patentblatt 81/17**

(45) Bekanntmachung des Hinweises auf die Patenterteilung:
**16.03.83 Patentblatt 83/11**

(84) Benannte Vertragsstaaten:
**AT BE CH DE FR GB LI NL**

(56) Entgegenhaltungen:
**DE-A-2 407 023**
**DE-A-2 642 606**
**GB-A-955 309**
**US-A-3 993 777**

(73) Patentinhaber: **Henkel Kommanditgesellschaft auf Aktien, Postfach 1100 Henkelstrasse 67, D-4000 Düsseldorf 1 (DE)**

(72) Erfinder: **Lauermann, Georg, Dr., Burscheider Weg 123, D-4021 Metzkausen (DE)**
Erfinder: **Koch, Ferdinand, Händelstrasse 36, D-4010 Hilden (DE)**

## Präparat zur Bekämpfung des Kannibalismus der Schweine

Im Rahmen der Aufstockung der Schweinebestände in der bäuerlichen Intensivtierhaltung hat sich eine Unart der Schweine entwickelt, die zu erheblichen wirtschaftlichen Verlusten in den Beständen führt, der sogenannte Kannibalismus. Im Zuge der strohlosen Haltung auf Spaltenböden ist dieses Problem noch deutlicher hervorgetreten. Mastschweine, die restriktiv gefüttert werden, entwickeln im Laufe der Mastperiode eine abartige Aggressivität gegenüber ihren Buchtengenossen und beginnen damit, diese zu beissen. Dabei entstehen im Schwanzbereich und an den Ohren tiefe Wunden, die durch Stallschmutz infiziert zu schwerwiegenden Erkrankungen und Ausfällen führen. Die Ursachen für den Kannibalismus sind noch nicht richtig aufgeklärt.

Es wird neuerdings empfohlen, vorbeugend im Ferkelalter den Schwanz zu amputieren, da dieser in der Hauptsache attackiert und angefressen wird. Die oberflächlich liegenden Schwanzarterien werden jedoch dabei häufig angerissen, wobei es zu unstillbaren Blutungen kommt. Auch einer Eröffnung und nachträgliche Infektion des letzten Endes des Wirbelkanals ist nicht selten, wodurch die Tiere dann bis zur Schlachtreife in der Nachhand vollständig gelämt sein können. Mastleistungen sind bei diesen Schweinen nicht mehr zu erzielen.

Vielfach wird angenommen, dass der Kannibalismus Ausdruck von sozialen Rangkämpfen ist. Angegriffene und vom Futtertrog abgebissene Schweine zeigen deutlich Todesangst und sondern dabei vermutlich auch Schweissgerüche ab, die den Angreifer zu weiteren Attacken ermuntern. Deshalb werden seit Jahren spezielle Duftstoffe verwendet, um die Angstgerüche zu überdecken und damit den Angreifer in der Suche des Opfers zu irritieren.

Diese Massnahme hat sich allerdings nicht bewährt. Derzeitig wird als Vorbeugungsmassnahme nur der Schwanz der Ferkel in den ersten Lebenstagen kupiert. Dem Kannibalismus kann jedoch auch damit nicht entscheidend begegnet werden, weil in derartig behandelten Beständen die Rangkämpfe blutig weitergehen. Bei amputierten Tieren mit Vorliebe an den Ohren.

Aus DE-A-2 407 023 sind Zusammensetzungen zur Bekämpfung von Kannibalismus und Kaudophagie bei Schweinen bekannt, die als Wirkstoff 8-Hydroxychinolin oder eines seiner Derivate enthalten. Hierbei handelt es sich jedoch um Stoffe, die physiologisch nicht unbedenklich sind bzw. dem Arzneimittelbereich zuzurechnen sind.

DE-A-2 642 606 betrifft ein Schneckenvertilgungsmittel, dass u.a. Benzyldiäthyl-(2,6-xylylcarbamoylmethyl)ammoniumbenzoat enthält. Dieses Mittel kommt als Granulat zur Anwendung und könnte – abgesehen von seiner Toxizität auf Grund des Gehaltes an Metaldehyd – für den erfindungsgemässen Zweck nicht eingesetzt werden.

Aus US-A-3 993 777 ist eine wässrige Komposition zur Verhinderung von Rindermastitis bekannt, die quaternäre Ammoniumverbindungen mit germicider Wirkung enthält. Diese Stoffe besitzen keine ausgesprochene Bitterwirkung und eignen sich daher nicht zur Bekämpfung des Kanibalismus der Schweine im Sinne der Erfindung.

Die Erfindung betrifft ein flüssiges Produkt zur Behandlung der gefährdeten Körperteile von Ferkeln und Schweinen, das durch seinen extrem bitteren Geschmack andere Artgenossen vom Beissen abschreckt.

Beansprucht wird demgemäss ein Präparat zur Bekämpfung des Kannibalismus der Schweine, gekennzeichnet durch folgende Zusammensetzung:

0,2–3,5 Gew.-% Benzyldiethyl- (2,6-xylylcarbamoylmethyl)-ammoniumben

5,0–25,0 Gew.-% o-Sulfo-benzoesäure-imid (Saccharin)

0,8–5,5 Gew.-% eines Celluloseethers als Verdickungsmittel

0,02–2,5 Gew.-% Glyoxal 40%ig

0,03–1,2 Gew.-% eines wasserlöslichen Farbstoffs

Rest Wasser

Als Verdickungsmittel zur Einstellung der Viskosität können handelsübliche Celluloseether eingesetzt werden, z.B. Methylcellulose oder Carboxymethylcellulose. Besonders bevorzugt wird Methylhydroxypropylcellulose. Die Viskosität des Produktes wird zweckmässig auf einen solchen Wert eingestellt, dass der Auslauf im DIN-Becher No. 4 bei 20 °C 500–600 Sekunden beträgt (DIN-Methode 53 211).

Benzyldiethyl-(2,6-xylylcarbamoylmethyl)-ammoniumbenzoat ist ein extrem bitter schmeckender Stoff, der auch unter dem Handelsnamen Bitrex [R] bekannt ist. Der ausserdem dazu gegebene Süssstoff Saccharin intensiviert den Bittergeschmack und verlängert die Haftung auf den Schleimhäuten.

Der wasserlösliche Farbstoff dient zur Kontrolle des Auftrags und der Haftung des Produktes auf den behandelten Stellen. Vorzugsweise wird Rhodamin B eingesetzt.

Die Wirkstofformulierung wird mit Wasser – vorzugsweise destilliertem oder demineralisiertem Wasser – auf 100 Gew.-% aufgefüllt.

Der bittere Geschmack des Präparates ist derartig abschreckend, dass den Schweinen das Beissen der Artgenossen vollständig verleidet wird. Schweine, die sich anschicken, den Schwanz anderer Tiere zum Beissen mit dem Rüssel zu untersuchen, spüren schon Sekunden später den extrem bitteren Geschmack und sind nach Kontakt mit dem Mittel längere Zeit beschäftigt, durch Kauen und Speicheln den Bitterstoff von der Zunge zu entfernen. Dieser haftet auch in geringsten Mengen sehr gut im Maul sowie auch auf der Haut des zu schützenden Tieres.

Toxikologisch ist das Produkt völlig unbedenklich, weil schon der Geschmack jegliche Aufnahme verhindert. Aus Gründen möglicher Geschmacksbeeinflussung des Fleiches soll das Produkt 3 Wochen vor dem Schlachten nicht mehr verwendet werden.

Zur Anwendung wird eine 1–3%ige wässrige Lösung des Präparates hergestellt. Die Applikation erfolgt zweckmässig mit einer Rückenspritze durch Aufsprühen auf die gefährdeten Körperteile der Tiere. Das Mittel kann aber auch mit einem Pinsel oder Schwamm oder dgl. aufgetragen werden. Behandelt werden in erster Linie die Schwanz- und Analregion sowie die Ohren der Schweine.

Bei starkem Auftreten von Kannibalismus ist die Behandlung zu wiederholen. Sie kann ohne erheblichen Arbeitsaufwand erfolgen. Die Zusammenstellung neu erworbener Schweine zu Mastgruppen sollte grundsätzlich unter dem Schutz des Bittermittels erfolgen. Dabei ist es nicht mehr erforderlich, vorbeugend die Schwänze zu kupieren.

Das Produkt eignet sich auch hervorragend für das Aufsprühen auf Geräte, Flächen, Käfigteile u.dgl., wo Tiere, z.B. Pferde gerne nagen und damit Stalleinrichtungen zerstören können.

Beispiel

0,7 Gew.-% Benzyldiethyl-(2,6-xylylcarbamoyl-methyl)-ammoniumbenzoat

20,0 Gew.-% Saccharin

2,0 Gew.-% Methylhydroxypropylcellulose (Culminal MHPC 1500 [R] der Firma Henkel)

0,5 Gew.-% Glyoxal

0,3 Gew.-% Rhodamin B

Rest Wasser

**Patentanspruch**

Präparat zur Bekämpfung des Kannibalismus der Schweine, gekennzeichnet durch folgende Zusammensetzung:

0,2–3,5 Gew.-% Benzyldiethyl-(2,6-xylylcarbamoylmethyl)-ammoniumbenzoat

5,0–25,0 Gew.-% o-Sulfo-benzoesäure-imid (Saccharin)

0,8–5,5 Gew.-% eines Celluloseethers als Verdickungsmittel

0,02–2,5 Gew.-% Glyoxal 40%ig

0,03–1,2 Gew.-% eines wasserlöslichen Farbstoffs

Rest Wasser

**Revendication**

Produit pour combattre le cannibalisme des porcs, caractérisé par la composition suivante:

0,2 à 3,5% en poids de benzoate de benzyldiéthyl-(2,6-xylylcarbamoylméthyl)-ammonium,

5,0 à 25,0% en poids d'imide o-sulfobenzoïque (saccharine),

0,8 à 5,5% en poids d'un éther cellulosique qui sert d'agent épaississant,

0,02 à 2,5% en poids de glyoxal à 40%,

0,03 à 1,2% en poids d'un colorant soluble dans l'eau,

complément: eau.

**Claim**

A preparation for controlling cannibalism in pigs, characterised by the following composition:

0.2 to 3.5% by weight of benzyldiethyl-(2,6-xylylcarbamoylmethyl)-ammonium benzoate

5.0 to 25.0% by weight of o-sulfobenzoic acid imide (saccharin)

0.8 to 5.5% by weight of a cellulose ether as thickener

0.02 to 2.5% by weight of glyoxal (40%)

0.03 to 1.2% by weight of a water-soluble dye remainder water.